# EUROPEAN PATENT APPLICATION

(11) **EP 2 264 179 A1**
(43) Date of publication of application: **22.12.2010**
(21) Application number: 09730818.3
(22) Date of filing: 09.04.2009
(51) Int. Cl.: C12P 19/04, A61K 31/715, A61K 35/74, A61P 3/10, A61P 43/00, C08B 37/00, C12N 1/20, A61K 31/7034, C07H 15/203

(54) **AMINOSUGAR COMPOUND AND PROCESS FOR PRODUCTION THEREOF**

(30) Priority: 11.04.2008 JP 2008102983
(71) Applicant: Astellas Pharma Inc., Tokyo 103-8411 (JP)
(72) Inventor: SASAMURA, Satoshi, Tokyo 103-8411 (JP); SASAMURA, Hiromi, Tokyo 103-8411 (JP); NISHIWAKI, Shinya, Tokyo 103-8411 (JP)
(74) Representative: HOFFMANN EITLE
(86) International application number: PCT/JP2009/057292
(87) International publication number: WO 2009/125819

(57) **Abstract**

Disclosed is a compound useful as an active ingredient for a pharmaceutical composition having an α-amylase-inhibiting activity, particularly a pharmaceutical composition for the treatment of diabetes. Studies have been made for discovering a compound having an α-amylase-inhibiting activity among the compounds produced by *Streptomyces sp.* Strain 6982 which is a ray fungus belonging to the genus *streptomyces,* and it is confirmed that an aminosugar compound has an α-amylase-inhibiting activity. The aminosugar compound has an α-amylase-inhibiting activity, and therefore can be used as a prophylactic or therapeutic agent for diabetes, obesity, NASH (non-alcoholic steatohepatitis), particularly as an ameliorating agent for postprandial blood glucose elevation.

SEQUENCE LISTING

<110> Astellas Pharma Inc.

<120> Aminosugar compound and production method thereof

<130> A09004

<150> JP 2008-102983

<151> 2008-4-11

<160> 1

<210> 1

<211> 1448

<212> DNA

<213> Streptomyces sp.

<400> 1
cttaacacat gcaagtcgaa cgatgaacca cttcggtggg gattagtggc gaacgggtga 60
gtaacacgtg ggcaatctgc cctgcactct gggacaagcc ctggaaacgg ggtctaatac 120
cggatactga tcatcttggg catccttgat gatcgaaagc tccggcggtg caggatgagc 180
ccgcggccta tcagcttgtt ggtgaggtaa cggctcacca aggcgacgac gggtagccgg 240
cctgagaggg cgaccggcca cactgggact gagacacggc ccagactcct acgggaggca 300
gcagtgggga atattgcaca atgggcgcaa gcctgatgca gcgacgccgc gtgagggatg 360
acggccttcg ggttgtaaac ctctttcagc agggaagaag cgaaagtgac ggtacctgca 420
gaagaagcgc cggctaacta cgtgccagca gccgcggtaa tacgtagggc gcaagcgttg 480
tccggaatta ttgggcgtaa agagctcgta ggcggcttgt cgcgtcggat gtgaaagccc 540
ggggcttaac cccgggtctg cattcgatac gggcaggcta gagttcggta ggggagatcg 600
gaattcctgg tgtagcggtg aaatgcgcag atatcaggag gaacaccggt ggcgaaggcg 660
gatctctggg ccgatactga cgctgaggag cgaaagcgtg gggagcgaac aggattagat 720
accctggtag tccacgccgt aaacggtggg cactaggtgt gggcaacatt ccacgttgtc 780
cgtgccgcag ctaacgcatt aagtgccccg cctggggagt acggccgcaa ggctaaaact 840
caaaggaatt gacgggggcc cgcacaagcg gcggagcatg tggcttaatt cgacgcaacg 900
cgaagaacct taccaaggct tgacatacac cggaaacact cagagatggg tgcccccttg 960
tggtcggtgt acaggtggtg catggctgtc gtcagctcgt gtcgtgagat gttgggttaa 1020
gtcccgcaac gagcgcaacc cttgtcccgt gttgccagca ggcccttgtg gtgctgggga 1080
ctcacgggag accgccgggg tcaactcgga ggaaggtggg gacgacgtca agtcatcatg 1140
ccccttatgt cttgggctgc acacgtgcta caatggccgg tacaatgagc tgcgataccg 1200
cgaggtggag cgaatctcaa aaagccggtc tcagttcgga ttggggtctg caactcgacc 1260
ccatgaagtc ggagtcgcta gtaatcgcag atcagcattg ctgcggtgaa tacgttcccg 1320
ggccttgtac acaccgcccg tcacgtcacg aaagtcggta acacccgaag ccggtggccc 1380

aaccccttgt gggagggagc tgtcgaaggt gggactggcg attgggacga agtcgtaaca 1440
aggtagcc 1448

## Description

### Technical Field

The present invention relates to an aminosugar compound that is useful as an active ingredient for a pharmaceutical composition, particularly a pharmaceutical composition for the treatment of diabetes.

### Background Art

Polysaccharides that have been ingested through a meal are partially digested by a saliva-derived α-amylase in the oral cavity and the stomach, and then most of the polysaccharides are digested by a pancreas-derived α-amylase in the duodenum and the jejunum, thereby being converted to disaccharides or oligosaccharides. These are hydrolyzed into monosaccharides by glucosidases represented by maltase and sucrase, which are localized in the microvillar membrane of the small intestine epithelium, and the monosaccharides are absorbed through the intestinal tract. The absorbed monosaccharides are transited into the blood, and when the blood glucose level increases, insulin is secreted from the pancreas so as to decrease the sugar release from the liver and also to increase the intake of sugar into the muscles or fatty tissues. Thereby, the increased blood glucose level is lowered, and the homeostasis is maintained.

However, in the pathological condition of diabetes, a patient is in the state of suffering from chronic failure of blood glucose control, such as postprandial hyperglycemia or interdigestive hyperglycemia, due to the hyposecretion of insulin secretion or insulin resistance (hypoactivity of insulin).

In recent years, it was verified by a large-scale clinical trial that correction of the postprandial hyperglycemia is important in the suppression of the onset and progress of diabetes complications. It has been proved that even a mild degree of postprandial hyperglycemia is an independent risk factor for cardiovascular deaths. Under such circumstances, the importance and necessity of medication treatment for postprandial hyperglycemia (for example, the state in which the blood glucose level measured 2 hours after meal is 200 mg/dL or higher) has come to be recognized.

Glucosidase inhibitors, which are digestive enzyme inhibitors, have been put to actual clinical use (for example, acarbose or voglibose) as therapeutic drugs for postprandial hyperglycemia. However, adverse side effects of the occurrence of symptoms in the digestive system (a sense of abdominal fullness, diarrhea, loose stools, flatulence, obdomial wind, and the like) caused by glucosidase inhibition have become a problem.

Furthermore, another example of a therapeutic drug for postprandial hyperglycemia (digestive enzyme inhibitory drug) is an α-amylase inhibitor. While inhibition of a glucosidase results in the side-production of large amounts of undigested oligosaccharides (disaccharides), inhibition of an α-amylase results in the by-production of only a small amount of undigested oligosaccharides (disaccharides), and therefore, it is expected that the α-amylase inhibitor can manifest the inhibition of glucose absorption without causing symptoms in the digestive system such as diarrhea.

Several aminosugar compounds having an α-amylase inhibitory activity have been reported.
For example, there has been reported a trestatin derivative (the following formula, provided that n in the formula represents 1 to 3) isolated from a actinomycetes belonging to the genus *Streptomyces* (Patent Literature 1).

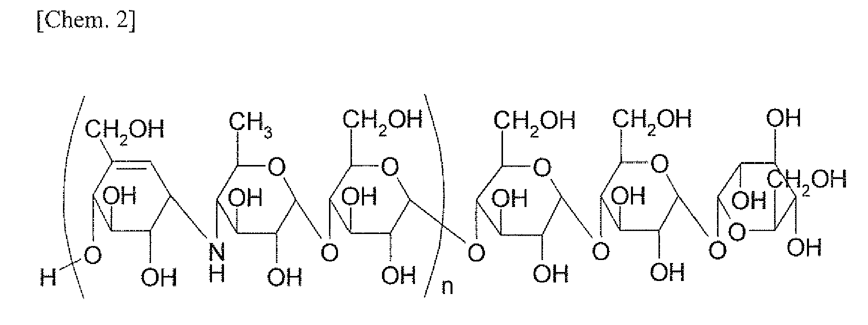

A malto-oligosaccharide compound having hexahydro-3,5,6-trihydroxy-1H-azepine as an essential skeleton has also been reported (Patent Literature 2). 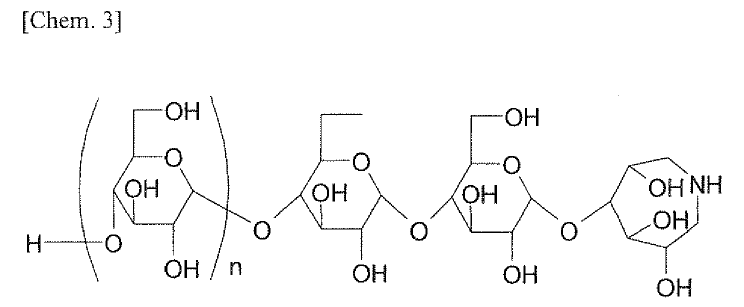 (wherein n represents 0 to 3; and X represents H or a hydrophobic group).

Furthermore, a compound having a reducing sugar structure at the terminals, in which n in the following formula is from 0 to 3, is known (Non-Patent Literature 1). 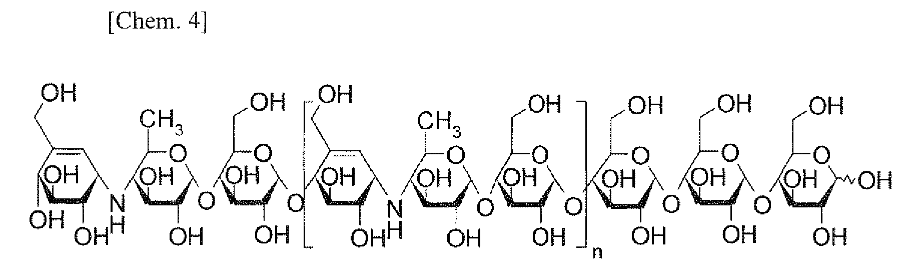 Acarviostatin IV03 (n=3), which is disclosed in the non-patent Literature, has a molecular formula of C₉₄H₁₅₆N₄O₆₄, which is identical with the compound of the invention wherein n = 4. However, this compound is a compound having a reducing sugar structure at the terminals and is therefore different in structure from the compound of the invention.

### [Citation List]

### [Patent Literature]

Patent Literature 1: JP-A-54-163511
Patent Literature 2: Specification of U.S. Patent No. 6596696 [Non-Patent Literature]

Non-Patent Literature 1: Carbohydrate Research, 343 (2008), 882-892

### Summary of the Invention

### Problem to Be Solved by the Invention

The invention provides a compound that is useful as an active ingredient for a pharmaceutical composition, particularly a pharmaceutical composition for the treatment of diabetes.

### Means for Solving the Problem

An α-amylase inhibitory drug needs to show stability in the digestive tract. Being unstable in the digestive tract implies that there is a possibility that the degradation product may be absorbed and may cause unexpected adverse side effects.
The present inventors made an extensive investigation of substances produced by microorganisms for the purpose of searching for a medicine, and as a result, the inventors found that strain 6982 of a actinomycetes belonging to the genus *Streptomyces* produces a compound having an excellent α-amylase inhibitory activity, thus completing the invention.

Thus, the present invention relates to a compound of formula (I) or a salt thereof; a composition comprising a compound of formula (I) or a salt thereof; a pharmaceutical composition comprising a compound of formula (I) or a salt thereof, and an excipient; and a process for production of a compound of formula (I) or a salt thereof.

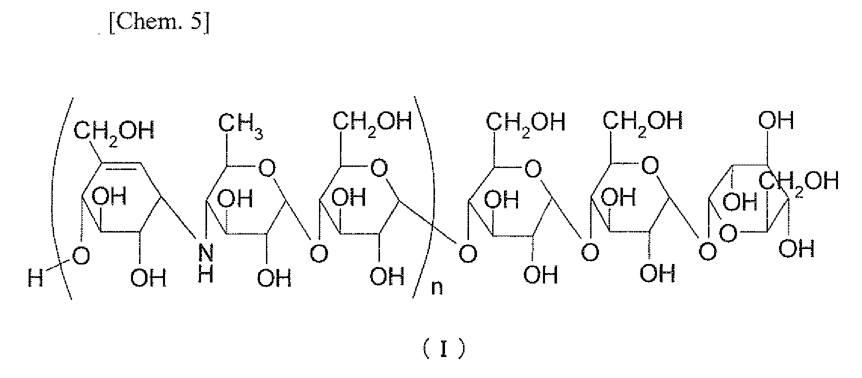

wherein n is 4, 5 or 6.

The present invention also relates to a compound having an α-amylase inhibitory activity and having a molecular formula of C₉₄H₁₅₆N₄O₆₄, C₁₁₃H₁₈₇N₅O₇₆ or C₁₃₂H₂₁₈N₆O₈₈ (provided that those compounds having a reducing sugar structure at the terminals are excluded), or a salt thereof, the compound being obtainable by culturing a microorganism belonging to the genus *Streptomyces* that produces a compound having an α-amylase inhibitory activity or a salt thereof in a medium, and recovering the compound or a salt thereof from the culture medium; and a composition comprising the compound or a salt thereof.
Moreover, the present invention relates to a pharmaceutical composition for the prevention or treatment of diabetes, comprising a compound of formula (I) or a salt thereof, i.e., an agent for treating diabetes, comprising a compound of formula (I) or a salt thereof.
Further, the present invention relates to use of a compound of formula (I) or a salt thereof, for the production of a pharmaceutical composition for the prevention or treatment of diabetes; use of a compound or a salt thereof, for the prevention or treatment of diabetes; and a method for the prevention or treatment of diabetes, the method comprising administering an effective amount of a compound of formula (I) or a salt thereof to a patient.

### Effect of the Invention

The compound of formula (I) or a salt thereof has an α-amylase inhibitory action and can be used as a prophylactic or therapeutic agent for diabetes, obesity, NASH (non-alcoholic steatohepatitis) and the like. The compound or a salt thereof is also expected to be able to inhibit the absorption of glucose without causing symptoms in the digestive system such as diarrhea, unlike the glucosidase inhibitors.

### Description of Embodiments

[FIG. 1] FIG. 1 shows the ¹H-NMR spectrum of compound A.
[FIG. 2] FIG. 2 shows the ¹³C-NMR spectrum of compound A.
[FIG. 3] FIG. 3 shows the ¹H-NMR spectrum of compound B.
[FIG. 4] FIG. 4 shows the ¹³C-NMR spectrum of compound B.
[FIG. 5] FIG. 5 shows the ¹H-NMR spectrum of compound C.
[FIG. 6] FIG. 6 shows the ¹³C-NMR spectrum of compound C.
[FIG. 7] FIG. 7 shows the blood glucose elevation suppressive action of the compound A under a load of carbohydrates. (A) shows the changes over time in the blood glucose level, and (B) shows the area-under-curve (AUC).
[FIG. 8] FIG. 8 shows the insulin elevation suppressive action of the compound A under a load of carbohydrates. (A) shows the changes over time in the insulin level, and (B) shows the AUC.
[FIG. 9] FIG. 9 shows the HPLC chromatogram of compound A.
[FIG. 10] FIG. 10 shows the HPLC chromatogram of compound B.
[FIG. 11] FIG. 11 shows the HPLC chromatogram of compound C.

### Embodiment for Carrying Out the Invention

For example, in the compound (I) of the invention, a compound in which n is 4 is represented by the following formula.

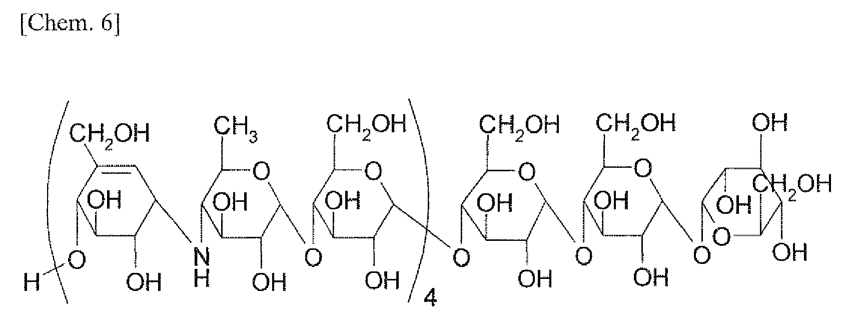

The compound of formula (I) can have geometrical isomers. In the present specification, the compound of formula (I) is described only in one form of the isomers, but the present invention includes the other isomers and includes isolated single examples of the isomers as well as mixtures thereof.
Furthermore, the compound of formula (I) can have optical isomers based on asymmetric carbon atom(s). The invention includes isolated single examples of the optical isomers of the compound of formula (I) as well as mixtures thereof.

The invention also includes pharmaceutically acceptable prodrugs of the compound represented by the formula (I). A pharmaceutically acceptable prodrug is a compound having a group that can be converted to an amino group, a hydroxyl group or the like through solvolysis or under physiological conditions. Examples of the group forming a prodrug include the groups described in Prog. Med., 5, 2157-2161 (1985) or "Pharmaceutical Research and Development" (Hirokawa Publishing Company, 1990), Vol. 7 Drug Design, 163-198.

A salt of the compound of formula (I) is a pharmaceutically acceptable salt of the compound of formula (I), and optionally forms an acid addition salt. Specific examples include the acid addition salts with inorganic acids such as hydrochloric acid, hydrobromic acid, hydroiodic acid, sulfuric acid, nitric acid and phosphoric acid; and with organic acids such as formic acid, acetic acid, propionic acid, oxalic acid, malonic aid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid and glutamic acid.

The invention also includes various hydrates or solvates and crystal polymorphs of the compound of formula (I) and salts thereof. The invention also includes compounds labeled with various radioactive isotopes or non-radioactive isotopes.

In the present specification, the "compound having reducing sugars at the terminals" is not particularly limited, but examples thereof include compounds of natural product and the like in which sugars having an aldehyde group (or ketone group) that can be free is present at the terminals, such as acarbose, acarviostatin 103, acarviostatin II03, acarviostatin III03 and acarviostatin IV03.

### (Process for production)

The compound of the invention can be produced by using a microorganism which belongs to the genus *Streptomyces* and is capable of producing the compound or a pharmaceutically acceptable salt thereof. A preferable example of such a microorganism is *Streptomyces* sp. strain 6982, which belongs to the genus *Streptomyces* and has been isolated from the soil collected at Iriomote Island in Okinawa Prefecture. The mycological properties of the subject strain are as follows.

*Streptomyces* sp. strain 6982 was isolated from the soil sample collected at Iriomote Island in Okinawa Prefecture. The medium and method for investigating the morphology, culturing properties and physiological properties of the subject strain mainly conform to Shirling, E.B. and D. Gottlieb: Methods for characterization of Streptomyces species. Int. J. Syst. Bacteriol. 16, 313-340, 1966, and Waksman, S.A.: The Actinomycetes Vol. 2: Classification, identification and description of genera and species: The Williams and Wilkins Co., Baltimore, 1961. Observation was made after culturing the strain at a culture temperature of 30°C and for a time period for culture of 14 days.

The observation of morphology was carried out by culturing the strain in a yeast extract-starch agar medium and then observing the strain under an optical microscope and a scanning electron microscope. The yeast extract-starch agar was prepared by preparing a solution containing 2.0 g of powdered yeast extract S (manufactured by Wako Pure Chemical Industries, Ltd.), 10 g of soluble starch, and 16 g of agar in 1 L of tap water, adjusting to pH = 7.2 with a 1 M aqueous solution of NaOH, and then sterilizing the solution in an autoclave. The growth temperature was determined with yeast extract-starch agar. The utility of the carbon source was determined in Pridoham and Gottlieb's medium (Pridoham, T.G. and D. Gottlieb: The utilization of carbon compounds by some Actinomycetales as an acid for species determination: J. Bacteriol. 56:107-114, 1948).

The color names were taken from "Methuen Handbook of Colour" (Komerup, A. and J.H. Wanscher: Methuen Handbook of Colour, Methuen, London, 1978).

The analysis of amino acids in the cell wall conformed to the method by Becker et al. (Becker, B., M.P. Lechevalier, R.E. Gordon and H.A. Lechevalier: Rapid differentiation between Nocardia and Streptomyces by paper chromatography of whole-cell hydrolysates: Appl. Microbiol. 12, 421-423, 1964).

The base sequence of 16S rDNA was determined according to the method by Nakagawa et al. (Nakagawa Yasuyoshi, and Kawasaki Hiroko, Classification and Identification of Actinomycetes, pp. 83-117, 2001, edited by the Society for Actinomycetes Japan: Tokyo, Business Center for Academic Societies Japan).

The homology search was carried out by using the FASTA search in the website of the National Institute of Genetics:
(a) http://www.ddbj.nig.ac.jp;
(b) D.J. Lipman, W.R. Pearson: Rapid and sensitive protein similarity searches, Science, 227, 1435-1.441 (1985); and
(c) W.R. Pearson, D.J. Lipman: Improved tools for biological sequence comparison, Proc. Natl. Acad. Sci. USA, 85, 2444-2448 (1988).
The base sequence of 16S rDNA of a standard strain was obtained from the database of the National Institute of Genetics (http://www.ddbj.nig.ac.jp).

The phylogenic tree was produced by a neighbor-binding method using Clustal W package (Clustal W Thompson, J.D., Higgins, D.G. and Gibson, T.J.: CLUSTAL W: improving the sensitivity of progressive multiple sequence alignment through sequence weighting, position-specific gap penalties and weight matrix choice. Nucleic Acids Res. 22, 4673-4680, 1994).

The DNA homology of *Streptomyces* sp. strain 6982 and its closely related strains can be confirmed by the method by Ezaki et al. shown in the following documents.
(a) Ezaki, T., Hashimoto, Y., Takeuchi, T., Yamamoto, H., Liu, S.-L., Matsui, K & Yabuuchi, E., J. Clin. Microbiol., 26, (1988) 1708-1713; and
(b) Ezaki, T., Hashimoto, Y. & Yabuuchi, E., Int. J. Syst. Bacteriol., 39, (1989) 224-229.

In the present specification, the "closely related strain" means a strain having at least 97% homology of the base sequence of 16S rDNA with *Streptomyces* sp. strain 6982. It is known that if the homology of the base sequence of 16S rDNA is less than 97%, those strains are determined as different species.
Stackebrandt, E. & Goebel, B.M., Int. J Syst Bacteriol., 44, (1994) 846-849.

### (1) Morphological features

The substrate mycelium was developed well and branched irregularly. The aerial mycelium exhibited an imperfect helical form, and was formed into 10 or more fragmented spore chains. The spores had smooth surfaces, an elliptical shape and a size of 1.2 × 1.0 µm. Sclerotic granules, sporangia, fragmentation of substrate mycelium and motile spores were not observed.

### (2) Cultural properties

The aerial mycelium exhibited good epiphytism on yeast extract-starch agar, yeast extract-malt extract agar, oatmeal agar, inorganic salt-starch agar, and glycerin-asparagine agar, and weak epiphytism was recognized on tyrosine agar. Aerial mycelium epiphytism was not recognized on peptone-yeast extract-iron agar. The color of the aerial mycelium was grayish brown or brownish gray. The color of the reverse side of growth was yellowish brown, brownish beige, grayish yellow, pale yellow, brownish orange, and pale orange. Production of melanoid pigments was not recognized on tryptone-yeast extract medium and peptone-yeast extract-iron agar. Production of soluble pigments was not recognized. The somatic pigments did not change with the pH.

The growth states on these various media are shown in Table 1. The abbreviations have the following meanings. G: growth, A: aerial mycelium, R: color of reverse side of growth, S: soluble pigment

**[Table 1]**

| Medium | Growth state | |
|---|---|---|
| Yeast extract-malt extract agar (ISP-2) | G | Good |
| | A | Rich, filamentous, grayish brown (7E3) |
| | R | Yellowish brown (5D6) |
| | S | None |
| Oatmeal agar (ISP-3) | G | Good |
| | A | Rich, filamentous, brownish gray (6E2) |
| | R | Brownish beige (6E3) |
| | S | None |
| Inorganic salt-starch agar (ISP-4) | G | Good |
| | A | Rich, brownish gray (6E2) |
| | R | Grayish yellow (4B5) |
| | S | None |
| Glycerin-asparagine agar (ISP-5) | G | Good |
| | A | Rich, filamentous, brownish gray (7E2) |
| | R | Pale yellow (4A3) |
| | S | None |
| Peptone-yeast extract-iron agar (ISP-6) | G | Medium degree |
| | A | None |
| | R | Brownish orange (5C6) |
| | S | None |
| Tyrosine agar (ISP-7) | G | Good |
| | A | Pale white |
| | R | Pale orange (5A3) |
| | S | None |

### (3) Cell wall type

The whole-cell degradation product was analyzed, and as a result, the presence of LL-diaminopimelic acid as an amino acid was confirmed.

### (4) Physiological properties

The utility of D-glucose, sucrose, D-xylose, D-fructose, L-rhamnose, raffinose, L-arabinose, inositol and D-mannitol was positive. Table 2 shows the physiological characteristics of *Streptomyces* sp. strain 6982.

**[Table 2]**

| Condition | | Characteristics |
|---|---|---|
| Growth temperature (°C) | | 12-45 |
| Optimal growth temperature (°C) | | 28-43 |
| Production of melanoid pigment | | - |
| Production of soluble pigment | | - |
| Utility of carbon source | | |
| | D-Glucose | ++ |
| | Sucrose | + |
| | D-Xylose | ++ |
| | D-Fructose | + |
| | L-Rhamnose | + |
| | Raffinose | ++ |
| | L-Arabinose | ++ |
| | Inositol | + |
| | D-Mannitol | + |

### (5) Analysis based on 16S rDNA base sequence

The 16S rDNA partial base sequence of *Streptomyces* sp. strain 6982 is shown in the attached Sequence Listing. As a result of the homology search, *Streptomyces glaucescens* strain DSM40716 (Accession No: X79322) having 99.2% homology was the most closely related strain. The homology with *Streptomyces albus* strain NBRC 13014 (Accession No: AB184257), which is a standard strain of the standard species of the genus *Streptomyces,* was 96.2%. Furthermore, in the phylogenic tree produced based on the 16S rDNA partial base sequence, the strain formed identical clusters with the various species of *Streptomyces.*

### (6) Identification

From the results of the morphological observation, chemical analysis and analysis based on the 16S rDNA base sequence with reference to the following documents (a) to (d), it has been concluded that the subject strain belongs to the genus *Streptomyces.*
(a) Euzeby, J.P.: List of bacterial names with standing in nomenclature: a folder available on the internet. Int. J. Syst. Bacteriol., 1997, 47, pp. 590-592.
(b) Waksman, S.A., et al.: The nomenclature and classification of the actinomycetes. Journal of Bacteriology, 1943, 46, pp. 337-341.
(c) Williams, S.T.: Bergey's Manual of Systematic Bacteriology, Vol. 4. 1989.
(d) Zhang, Z. et al.: A proposal to revive the genus Kitastospora, Int. J. Syst. Bacteriol., 1997, 47, pp. 1048-1054.

Thus, the subject strain was designated as *Streptomyces* sp. strain 6982. The subject strain was deposited internationally at the International Patent Organism Depositary (IPOD) National Institute of Advanced Industrial Science and Technology, under the Accession Number FERM BP-10802 (Date of deposit: March 22, 2007).
Since microorganisms undergo artificial or natural mutations, *Streptomyces* sp. strain 6982 of the invention includes, in addition to the microorganism isolated from nature, the strains obtained by artificially mutating the naturally isolated strain with ultraviolet radiation, X-rays, or chemicals, and natural mutants thereof.

The amino sugar derivative of the invention can be obtained by inoculating a microorganism which belongs to the genus *Streptomyces* and is capable of producing a compound having an α-amylase activity, preferably a microorganism capable of producing the relevant aminosugar derivative, and more preferably *Streptomyces* sp. strain 6982, into a medium containing nutrient sources, and aerobically growing the microorganism.

The medium used in the culture may be any medium in which the microorganism used can grow, and a synthetic medium, a semi-synthetic medium or a natural medium can be used.

As the nutrients, it is desirable to use the nutrient sources that can be utilized by the strain of the invention. Examples of the nutrient sources include, as nitrogen sources, inorganic or organic nitrogen sources such as soybean flour, defatted soybean flour, peptone, meat extract, corn steep liquor, cotton seed flour, peanut flour, soy flour, yeast extract, dry yeast, NZ-amine, hydrolytic product of casein, fish powder, sodium nitrate, and ammonium nitrate. Examples of carbon sources include carbohydrates such as starches such as potato starch and corn starch, molasses, dextrin, sucrose, glucose, maltose, trehalose, fructose, xylose, rhamnose, mannitol and glycerin, or fats. Preferable examples include starches and soybean flour.

Furthermore, sulfates, chlorates, nitrates, phosphates, carbonates and the like of Na, K, Mg, Ca, Zn and Fe and the like are added as metal salts if necessary, and preferable examples include calcium carbonate and/or sodium chloride. Moreover, if necessary, conventionally known amino acids, growth promoting compounds such as methyl oleate, lard oil, silicone oil and surfactants, or defoamants are appropriately used. In addition to these, any substances that can be used by the producing bacteria and are helpful to the production of the compound of the invention, can be used as desired.

Culture may be carried out in the same manner as in the culture for the production of general antibiotic substances, and the culturing method may be either solid culture or liquid culture. In the case of liquid culture, any of standing culture, shaking culture and stirring culture may be carried out, and for example, aeration-agitation culture may be carried out. In regard to the culturing conditions, the culturing temperature that can be appropriately applied may be a temperature at which the producing strain can grow and produce the compound of the invention, that is, in the range of 15 to 42°C, and a temperature of about 20 to 30°C is preferable, and a temperature of 23 to 27°C is more preferable. A pH that can be appropriately applied is in the range of 4 to 9, and pH 6 to 8 is preferable. The culturing time may vary with various conditions, and usually a time period in the range of 1 to 30 days can be appropriately applied, and a time period of 4 to 10 days is preferable.

In order to isolate the desired compound from the culture, conventional means for extraction, separation and purification that are used upon the isolation of metabolic products of microorganisms can be appropriately used. The substance in the culture is obtained by filtering the culture liquid directly, or filtering after centrifuging the culture liquid or adding a filtration aid to the culture, and thus a filtrate is obtained. At this time, an organic solvent such as acetone, MeOH, EtOH, or MeCN may be added to the culture liquid, or hydrochloric acid or the like may be added for pH adjustment, if desired. Furthermore, the substance can be separated by bringing the filtrate into contact with an appropriate carrier to adsorb the produced substance in the filtrate, and then eluting the substance with an appropriate solvent. For example, the filtrate is brought into contact with a porous adsorption resin such as AMBERLITE (registered trademark) XAD2, DIAION (registered trademark) HP20, DIAION CHP20P, or DIAION SP850, and thereby the substance is adsorbed. Subsequently, a liquid mixture of water and an organic solvent such as acetone, MeOH, EtOH or MeCN is used to elute the substance. Hydrochloric acid or the like may also be added for pH adjustment, if desired. In this case, a fraction containing the substance may be obtained efficiently by gradually or continuously increasing the mixing ratio of the organic solvent from low concentration to high concentration.

The compound of the invention was purified by adding a base, in order to eliminate any compound that is unstable and is difficult to isolate as a single product, such as a reducing sugar compound. Furthermore, the compound was repeatedly purified so as to secure the purity for a medicine.

The compound of formula (I) is isolated and purified as a free compound, a salt thereof, a hydrate, a solvate or a crystal polymorphic substance. A salt of the compound of formula (I) can be produced by subjecting the compound to a salt-producing reaction of a conventional method. Isolation and purification are carried out by applying conventional chemical operations such as extraction, fractionated crystallization, and various fractionated chromatography techniques.

According to the present specification, the term "recovery" means an operation carried out to obtain the compound (I) of the invention as a simple substance or as a composition, and includes "purification" or "isolation".

According to the present specification, the "purification" includes operations carried out for the purpose of "isolating" the compound of the invention, and the compound of the invention may be "isolated" by "purification".

Various isomers can be produced by selecting appropriate starting compounds, or can be separated by using the differences in the physicochemical properties between isomers. For example, an optical isomer may be obtained by a general optical resolution method for racemic bodies (for example, fractional crystallization induced by a diastereomer salt with an optically active base or acid, chromatography using a chiral column or the like).

According to the present specification, the term "chromatography" means the chromatography technique generally used in analytical chemistry, and examples include, but not limited to, gas chromatography (GC), liquid chromatography (LC), and supercritical fluid chromatography (SFC).

According to the present specification, the "liquid chromatography (LC)" means the generic name for chromatographic techniques using liquid in the mobile phase, and examples include, but not limited to, medium performance liquid chromatography and high performance liquid chromatography (HPLC).

A composition containing the compound of formula (I) or a salt thereof means a composition obtainable in the stage of purifying or isolating the compound of formula (I), and means a composition that can be used as a production source for a medicine. An example of the composition is, but not limited to, a mixture of one kind or two or more kinds of the compound of formula (I) or a salt thereof with an active ingredient such as acarviostatin IV03, or with other components. The subject composition contains the compound of formula (I) or a salt thereof at a proportion of, but not limited to, about 80% or more, preferably 85% or more, more preferably 90% or more, even more preferably 95% or more, and still more preferably 98% or more, in terms of area percentage as obtained by an analysis by chromato graphy.

A pharmaceutical composition containing one kind or two or more kinds of the compound of formula (I) or a salt thereof as active ingredients, can be prepared according to a conventionally used method, using the excipients that are conventionally used in this area that is, excipients for medicaments, carriers for medicaments or the like.
Administration may be carried out either by oral administration by means of tablets, pills, capsules, granules, powders or liquids, or by parenteral administration by means of intraarticular, intravenous or intramuscular injections, suppositories, eye drops, eye ointments, transdermal liquids, ointments, transdermal patches, transmucosal liquids, transmucosal patches, inhalants or the like.

As solid compositions for oral administration, tablets, powders, granules and the like are used. In these solid composition, one kind or two or more kinds of active ingredients are mixed with at least one inert excipient, for example, lactose, mannitol, glucose, hydroxypropyl cellulose, microcrystalline cellulose, starch, polyvinylpyrrolidone, and/or magnesium metasilicate aluminate. The composition may contain inert additives, for example, a lubricating agent such as magnesium stearate, a disintegrant such as carboxymethylstarch sodium, a stabilizer, and a dissolution aid, according to a conventional method. The tablets or pills may be coated with a sugar coating or a film of a gastric or enteric substance, if necessary.

Examples of the liquid composition for oral administration include pharmaceutically acceptable emulsions, solutions, suspensions, syrups elixirs, and the like, and the liquid composition contains a generally used inert diluent, for example, purified water or EtOH. The liquid composition may also contain, in addition to the inert diluent, auxiliary agents such as a solubilizer, a wetting agent and a suspending agent, as well as a sweetening agent, a flavoring agent, a fragrance and a preservative.

The injection for parenteral administration contains a sterile aqueous or non-aqueous solution, suspension or emulsion. Examples of an aqueous solvent include distilled water for injection, and physiological saline. Examples of a non-aqueous solvent include propylene glycol, polyethylene glycol, plant oils such as olive oil, alcohols such as EtOH, Polysorbate 80 (name according to the Japanese Pharmacopoeia), and the like. Such a composition may further contain an isotonic agent, a preservative, a wetting agent, an emulsifier, a dispersant, a stabilizer or a dissolution aid. These compositions are sterilized by, for example, filtration through a bacteria-retaining filter, incorporation of a bactericide, or irradiation. Furthermore, these compositions may be used by preparing a sterile solid composition, and dissolving or suspending the solid composition in sterilized water or a sterilized solvent for injection prior to use.

Agents for external use include an ointment, a plaster, a cream, a jelly, a PAP, a spray, a lotion, an eye drop, an eye ointment, and the like. These agents contain generally used ointment bases, lotion bases, aqueous or non-aqueous liquids, suspensions emulsions and the like. Examples of the ointment or lotion bases include polyethylene glycol, propylene glycol, white petrolatum, bleached beeswax, polyoxyethylene hydrogenated castor oil, glycerin monostearate, stearyl alcohol, cetyl alcohol, lauromacrogol, sorbitan sesquioleate and the like.

For the transmucosal preparations such as an inhalant and an intranasal agent, solid, liquid or semisolid preparations are used, and the preparations can be produced according to conventionally known methods. For example, the preparation may appropriately contain known excipients, a pH adjusting agent, a preservative, a surfactant, a lubricating agent, a stabilizer, a thickening agent and the like. The preparations can be administered by using an appropriate device for inhalation or in sufflation. For example, the compound can be administered alone, or as a powder of a prescribed mixture, or as a solution or suspension in combination with a pharmaceutically acceptable carrier, by using a known device such as a metered dose inhalation device, or a sprayer. A dry powder inhaler or the like may be a device for single administration or multiple administrations, and a dry powder or powder-containing capsules can be used. Alternatively, the preparation may be in the form of an appropriate ejection preparation, for example, a pressurized aerosol spray using a suitable gas such as chlorofluoroalkane, hydrofluoroalkane or carbon dioxide.

Typically, in the case of oral administration, the daily dose is suitably about 0.01 to 100 mg/kg, and preferably 0.1 to 10 mg/kg, per body weight, and this dose is administered once or in 2 to 4 divided portions. Furthermore, in the case of a transmucosal preparation, a dose of about 0.001 to 100 mg/kg per body weight is administered once or in several divided portions a day. The dose is appropriately determined in accordance with the individual cases, in consideration of symptoms, age, gender and the like.

The compound of formula (I) can be used together with various therapeutic agents or prophylactic agents for diseases against which the compound of formula (I) is believed to exhibit effectiveness. The combined preparation may be administered simultaneously, or separately but continually, or with a desired time interval. A preparation for simultaneous administration may be a mixed preparation or may be in separate preparations.

### EXAMPLES

Hereinafter, the process for producing the compound of formula (I) will be described in more detail based on Examples. The invention is not intended to be limited to the compounds described in the following Examples. Furthermore, the process for production of the compound of formula (I) is not intended to be limited to the specific production processes of the Examples shown below, and the compound of formula (I) can also be produced according to a combination of these production processes, or according to a method obvious to those ordinarily skilled in the art.

The following abbreviations may be used in the present specification.
EtOH: ethanol
MeCN: acetonitrile
MeOH: methanol
NaCl: sodium chloride
NaOH: sodium hydroxide
TFA: trifluoroacetic acid

Hereinafter, the invention will be described in more detail based on Examples, but the invention is not at all intended to be limited to these Examples.

### Example 1

### (Culture)

A seed medium was prepared by dispensing 30 mL each of a medium (pH = 7.0) containing 20 g of a soluble starch, 10 g of Pinedex #3 (manufactured by Matsutani Chemical Industry Co., Ltd.), 20 g of a soybean flour and 1 L of distilled water, into erlenmeyer flasks each having a capacity of 100 mL, and sterilizing the medium in an autoclave at 121°C for 30 minutes.
One platinum loop of a slant culture product of *Streptomyces* sp. strain 6982 was inoculated into this seed medium, and the inoculated seed medium was subjected to shaking culture for 3 days at 30°C.
A production medium was prepared by dispensing 30 mL each of a medium (pH = 7.0) containing 100 g of a soluble starch, 60 g of a soybean flour, 2.5 g of NaCl, 2 g of CaCO₃ and 1 L of distilled water, into erlenmeyer flasks each having a capacity of 100 mL, and sterilizing the medium in an autoclave at 121°C for 30 minutes.
The seed culture medium obtained as described above was inoculated into each flask of this production medium, in an amount of 2 mL each, and the resultant mixtures were subjected to shaking culture for 7 days at 25°C.

### Crude purification from culture medium

22 L of the culture liquid obtained by the culturing method described above was adjusted to pH = 3 with a 6 M aqueous HCl solution, and then the adjusted culture medium was suction filtered. The filtrate was adjusted to pH = 7 with a 6 M aqueous NaOH solution, and then the adjusted filtrate was applied to DIAION HP20 (resin amount 1.2 L). The column was washed with a 10% aqueous MeOH solution and then eluted with a 25% aqueous MeOH solution.

This active fraction was applied to DOWEX 50W X1 (manufactured by the Dow Chemical Company) cation exchange resin (resin amount 550 mL). The column was washed with distilled water, a 0.5 M aqueous NaCl solution and a 1 M aqueous NaCl solution, and then eluted with a 3 M aqueous NaCl solution.
Subsequently, the active fraction was applied to DAISOGEL SP-120-15/30-ODS-B (resin amount IL, manufactured by Daiso Co., Ltd.). The column was eluted with a 10% aqueous MeOH solution and a 15% aqueous MeOH solution to obtain compounds A and B, respectively, and the column was eluted with a 20% aqueous MeOH solution and a 25% aqueous MeOH solution to obtain compound C.

### (1) Purification and isolation of compound A

The active fraction containing compound A, which was obtained by purifying by ODS column chromatography, was applied to SP-120-15/30-ODS-B (resin amount 337 mL). The column was eluted with a 2% aqueous MeOH solution and a 3% aqueous MeOH solution (each containing 0.05% TFA).
This active fraction was adjusted to pH = 13 with a 6 M aqueous NaOH solution, and was applied to DIAION HP20SS (resin amount 500 mL)). The column was eluted with a 12.5% aqueous MeOH solution and a 15% aqueous MeOH solution (each containing 0.05 M NaOH).
The active fraction was adjusted to pH = 7 with a 6 M aqueous HCl solution, and was applied to DIAION HP20 (resin amount 200 mL) and the column was eluted with a 50% aqueous MeOH solution. The eluate was concentrated under reduced pressure, and then was freeze-dried to obtain 1.14 g of a powder.
200 mg of the powder was dissolved in water, and the solution was applied to DAISOPAK SP-120-5-ODS-BP (20 × 250 mm, manufactured by DAISO Co., Ltd.). The column was eluted with a 2% aqueous MeCN solution (containing 0.05% TFA).
This active fraction was adjusted to pH = 7 with a 6 M aqueous NaOH solution, and was applied to DIAION HP20 (resin amount 50 mL). The column was eluted with a 50% aqueous MeOH solution. The eluate was concentrated under reduced pressure, and was freeze-dried to obtain 136 mg of a powder.
The powder was dissolved in water, and the solution was applied to DAISOPAK SP-120-5-ODS-BP (20 × 250 mm). The column was eluted with a 2% aqueous MeCN solution (containing 0.05% TFA).
This active fraction was adjusted to pH = 7 with a 6 M aqueous NaOH solution, and was applied to DIAION HP20 (resin amount 30 mL). The column was eluted with a 50% aqueous MeOH solution.
The obtained active fraction was concentrated under reduced pressure, and was freeze-dried to obtain compound A (65 mg).

### (2) Purification and isolation of compound B

The fraction obtained by crude purification by ODS column chromatography was applied to SP-120-15/30-ODS-B (resin amount 337 mL). The column was eluted with a series of 3% to 7% aqueous MeOH solutions (each containing 0.05% TFA).
This active fraction was adjusted to pH = 13 with a 6 M aqueous NaOH solution, and was applied to DIAION HP20SS (resin amount 250 mL). The column was eluted with 15%, 17.5% and 20% aqueous MeOH solutions (each containing 0.05 M NaOH).
This active fraction was adjusted to pH = 7 with a 6 M aqueous HCl solution, and was applied to DIAION HP20 (resin amount 150 mL). The column was eluted with a 50% aqueous MeOH solution. The eluate was concentrated under reduced pressure, and then was freeze-dried to obtain 970 mg of a powder.
450 mg of the powder was dissolved in water, and the solution was applied to DAISOPAK SP-120-5-ODS-BP (20 × 250 mm). The column was eluted with a 2.2% aqueous MeCN solution (containing 0.05% TFA).
This active fraction was adjusted to pH = 7 with a 6 M aqueous NaOH solution, and was applied to DIAION HP20 (resin amount 115 mL). The column was eluted with a 50% aqueous MeOH solution. The eluate was concentrated under reduced pressure, and was freeze-dried to obtain 190 mg of a powder.
The powder was dissolved in water, and the solution was applied to DAISOPAK SP-120-5-ODS-BP (20 × 250 mm). The column was eluted with a 2.2% aqueous MeCN solution (containing 0.05% TFA)
This active fraction was adjusted to pH = 7 with a 6 M aqueous NaOH solution, and was applied to DIAION HP20 (resin amount 30 mL). The column was eluted with a 50% aqueous MeOH solution.
The obtained active fraction was concentrated under reduced pressure, and was freeze-dried to obtain compound B (129 mg).

### (3) Purification and isolation of compound C

The fraction obtained by crude purification by ODS column chromatography was applied to SP-120-15/30-ODS-B (resin amount 80 mL). The column was eluted with a 2% aqueous MeOH solution (each containing 0.05% TFA).
This active fraction was adjusted to pH = 13 with a 6 M aqueous NaOH solution, and was applied to DIAION HP20SS (resin amount 200 mL). The column was eluted with 15%, 17.5% and 20% aqueous MeOH solutions (each containing 0.05 M NaOH).
This active fraction was adjusted to pH = 7 with a 6 M aqueous HCl solution, and was applied to DIAION HP20 (resin amount 150 mL). The column was eluted with a 50% aqueous MeOH solution. The eluate was concentrated under reduced pressure, and then was freeze-dried to obtain 1 g of a powder.
The powder was dissolved in water, and the solution was applied to DAISOPAK SP-120-5-ODS-BP (20 × 250 mm). The column was eluted with a series of 2.2% to 3.2% aqueous MeCN solutions (each containing 0.05% TFA). This active fraction was adjusted to pH = 7 with a 6 M aqueous NaOH solution, and was applied to DIAION HP20 (resin amount 30 mL). The column was eluted with a 50% aqueous MeOH solution. The eluate was concentrated under reduced pressure, and was freeze-dried to obtain 236 mg of a powder.
184 mg of the powder was dissolved in water, and the solution was applied to DAISOPAK SP-120-5-ODS-BP (20 × 250 mm). The column was eluted with a 2.2% aqueous MeCN solution (containing 0.05% TFA).
This active fraction was adjusted to pH = 7 with a 6 M aqueous NaOH solution, and was applied to DIAION HP20 (resin amount 30 mL). The column was eluted with a 50% aqueous MeOH solution.
The obtained active fraction was concentrated under reduced pressure, and was freeze-dried to obtain compound C (91 mg).

### Physicochemical properties of compounds A, B and C

The compounds A, B and C obtained by extraction, separation and purification respectively had the following physicochemical properties.

### (1) Compound A

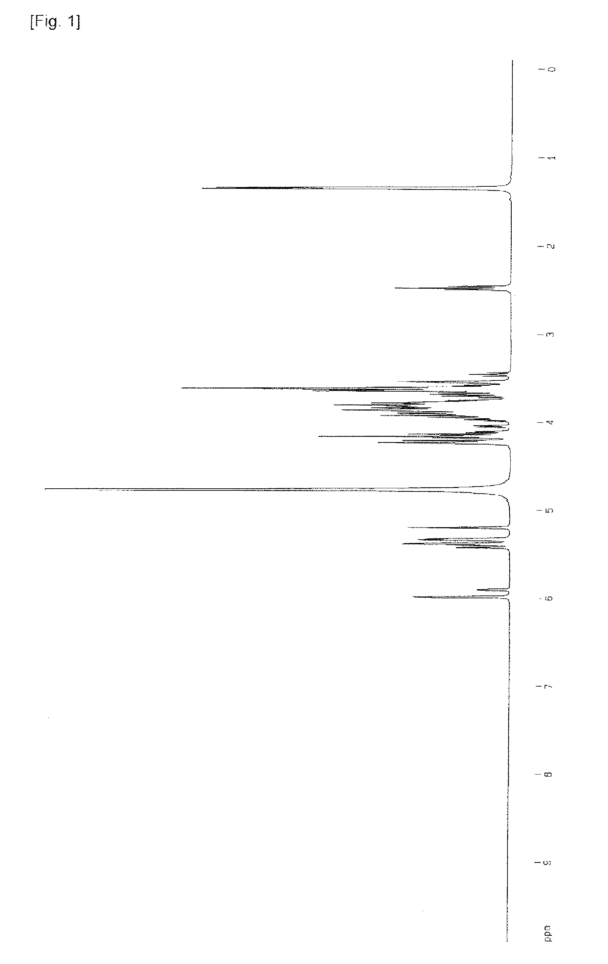

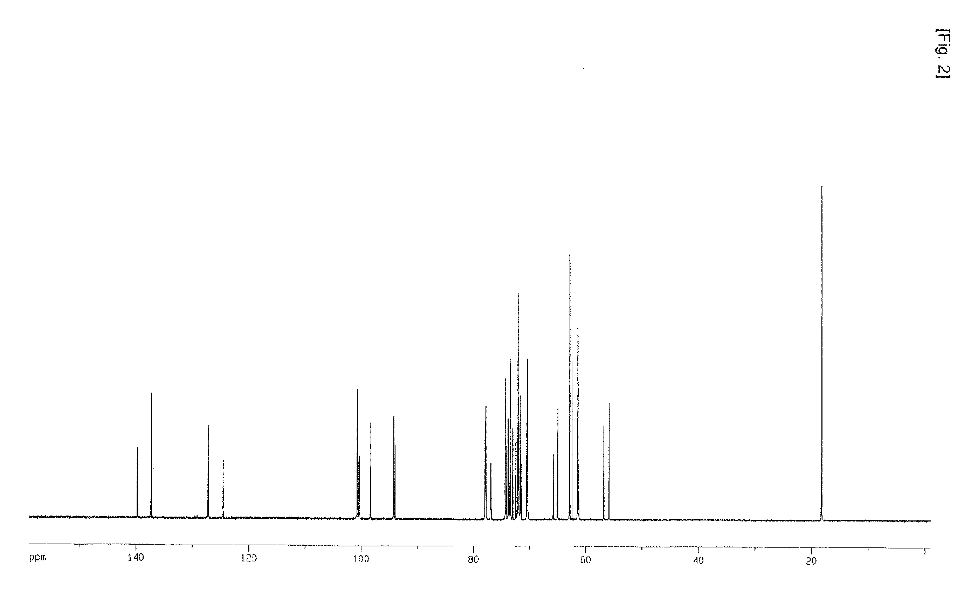

1) Color and shape: white powder
2) Classification by acidity, neutrality and basicity: basic
3) Specific rotation: [α]²³_{D} +157° (c = 0.5, H₂O)
4) Molecular formula: C₉₄H₁₅₆N₄O₆₄
5) High resolution TOF-mass spectrum:
   Found value [M+2H]²⁺ 1183.4624
   Theoretical value [M+2H]²⁺ 1183.4616
6) Elemental analysis: as C₉₄H₁₅₆N₄O₆₄ 14H₂O
   Calculated value: C 43.12, H 7.08, N 2.14,
   Found value: C 43.27, H 7.08, N 2.05
7) Solubility: highly soluble in water, DMF and DMSO, but slightly soluble in acetone, MeOH, EtOH and MeCN.
8) Ultraviolet absorption spectrum (solvent: water): shows end absorption
9) Infrared absorption spectrum (νₘₐₓ (KBr) cm⁻¹): 3330, 2925, 1655, 1385, 1150, 1040, 935
10) ¹H-NMR spectrum (500 MHz, D₂O): shown in FIG. 1
11) ¹³C-NMR spectrum (125 MHz, D₂O): shown in FIG. 2

### (2) Compound B

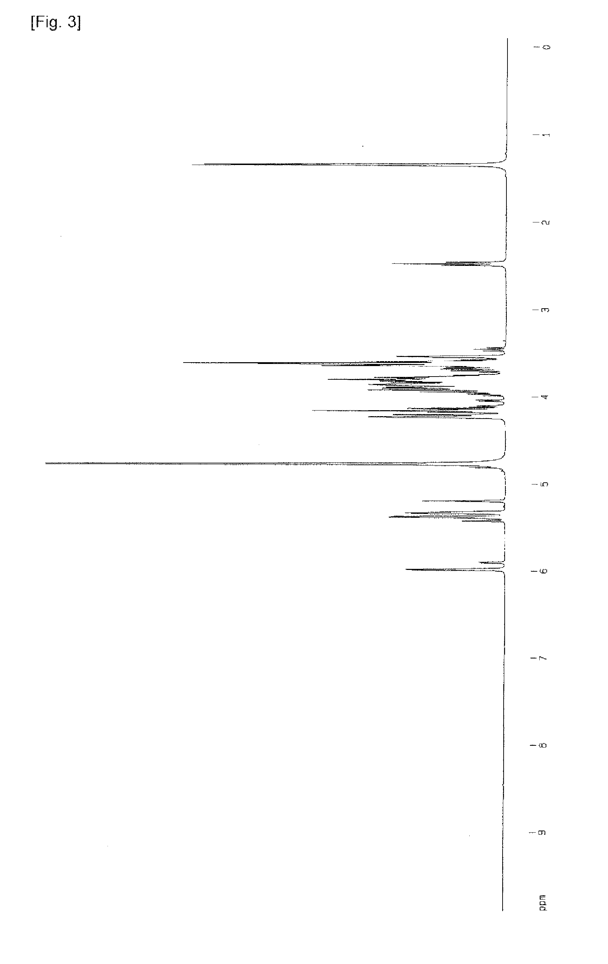

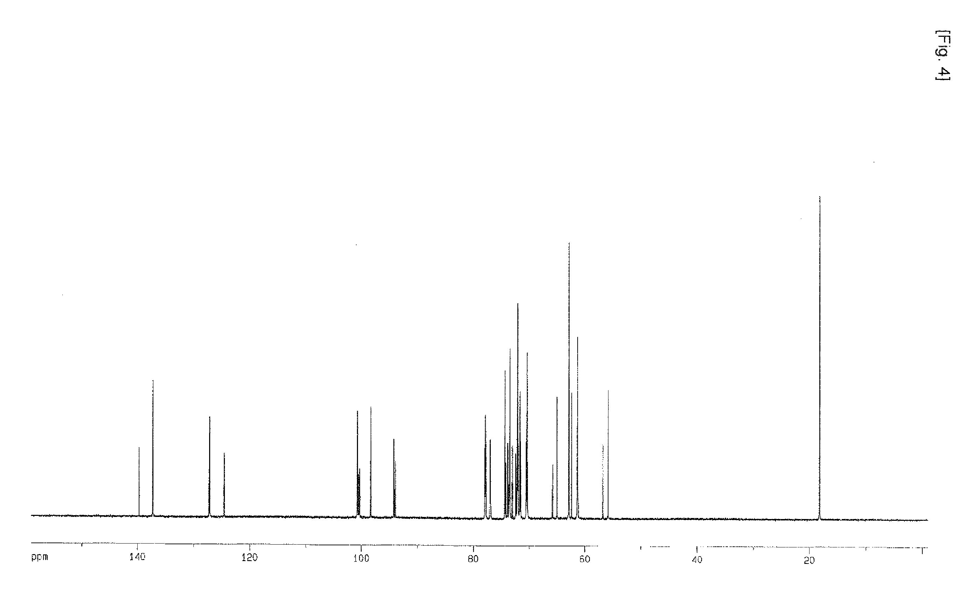

1) Color and shape: white powder
2) Classification by acidity, neutrality and basicity: basic
3) Specific rotation: [α]²³_{D} +153° (c = 0.5, H₂O)
4) Molecular formula: C₁₁₃H₁₈₇N₅O₇₆
5) High resolution TOF-mass spectrum:
   Found value [M+2H]²⁺ 1416.0548
   Theoretical value [M+2H]²⁺ 1416.0539
6) Elemental analysis: as C₁₁₃H₁₈₇N₅O₇₆ 16H₂O
   Calculated value: C 43.50, H 7.08, N 2.24,
   Found value: C 43.53, H 7.09, N 2.15
7) Solubility: highly soluble in water, DMF and DMSO, but slightly soluble in acetone, MeOH, EtOH and MeCN.
8) Ultraviolet absorption spectrum (solvent: water): shows end absorption
9) Infrared absorption spectrum (νₘₐₓ (KBr) cm⁻¹): 3365, 2930, 1640, 1385, 1150, 1045, 935
10) ¹H-NMR spectrum (500 MHz, D₂O): shown in FIG. 3
11) ¹³C-NMR spectrum (125 MHz, D₂O): shown in FIG. 4

### (3) Compound C

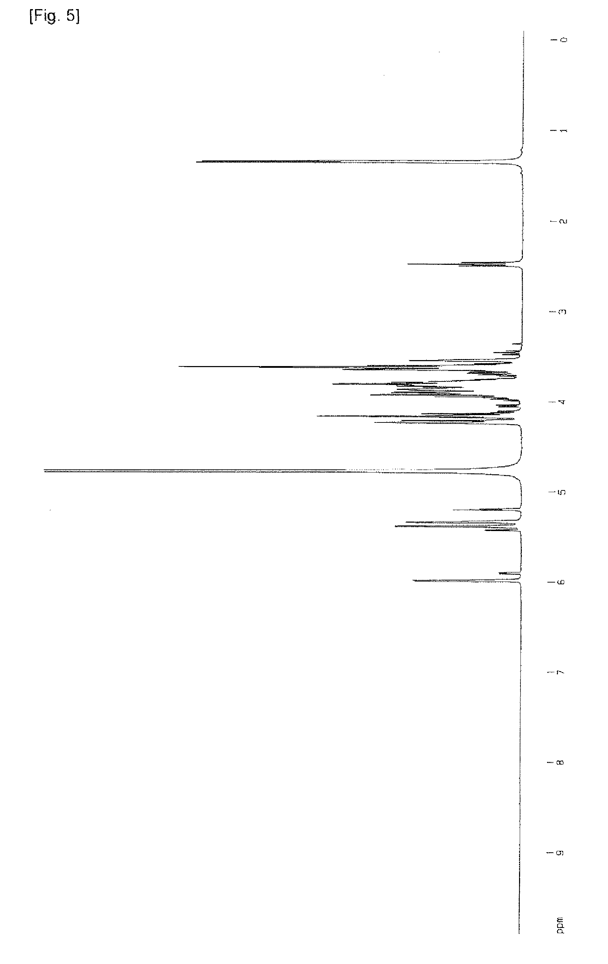

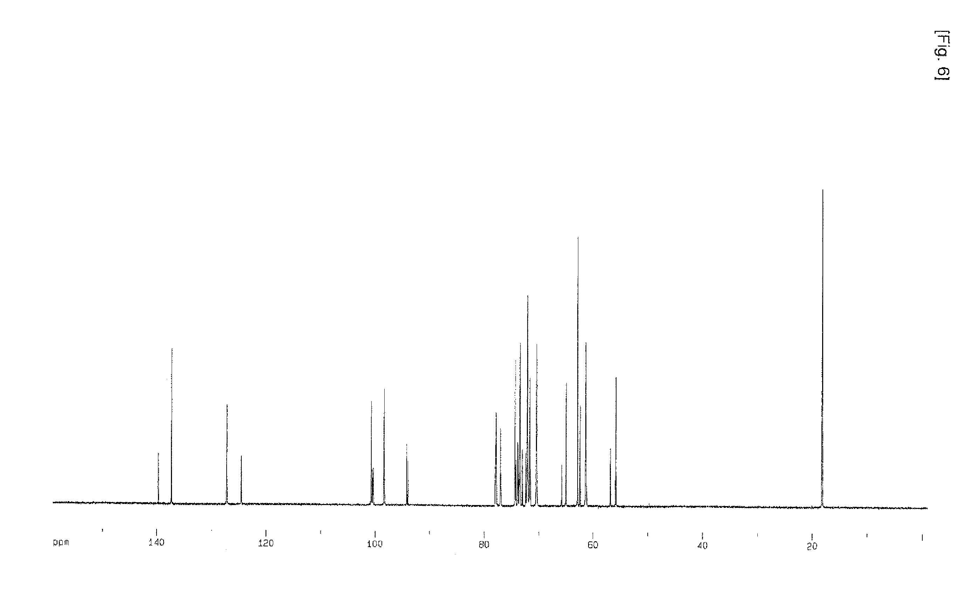

1) Color and shape: white powder
2) Classification by acidity, neutrality and basicity: basic
3) Specific rotation: [α]²³_{D} +172° (c = 0.5, H₂O)
4) Molecular formula: C₁₃₂H₂₁₈N₆O₈₈
5) High resolution TOF-mass spectrum:
   Found value [M+2H]²⁺ 1648.6467
   Theoretical value [M+2H]⁺ 1648.6462
6) Elemental analysis: as C₁₃₂H₂₁₈N₆O₈₈ 16H₂O
   Calculated value: C 44.22, H 7.03, N 2.34,
   Found value: C 44.23, H 7.09, N 2.35
7) Solubility: highly soluble in water, DMF and DMSO, but slightly soluble in acetone, MeOH, EtOH and MeCN.
8) Ultraviolet absorption spectrum (solvent: water): shows end absorption
9) Infrared absorption spectrum (νₘₐₓ (KBr) cm⁻¹): 3365, 2925, 1635, 1340, 1150, 1040, 935
10) ¹H-NMR spectrum (500 MHz, D₂O): shown in FIG. 5
11) ¹³C-NMR spectrum (125 MHz, D₂O): shown in FIG. 6
The chemical structures of the compounds A, B and C were determined as shown below, from the physicochemical properties described above. 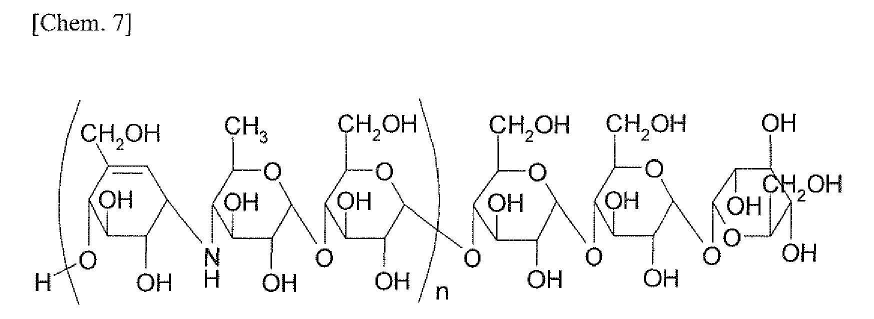 wherein compound A: n = 4; compound B: n = 5; and compound C: n = 6.

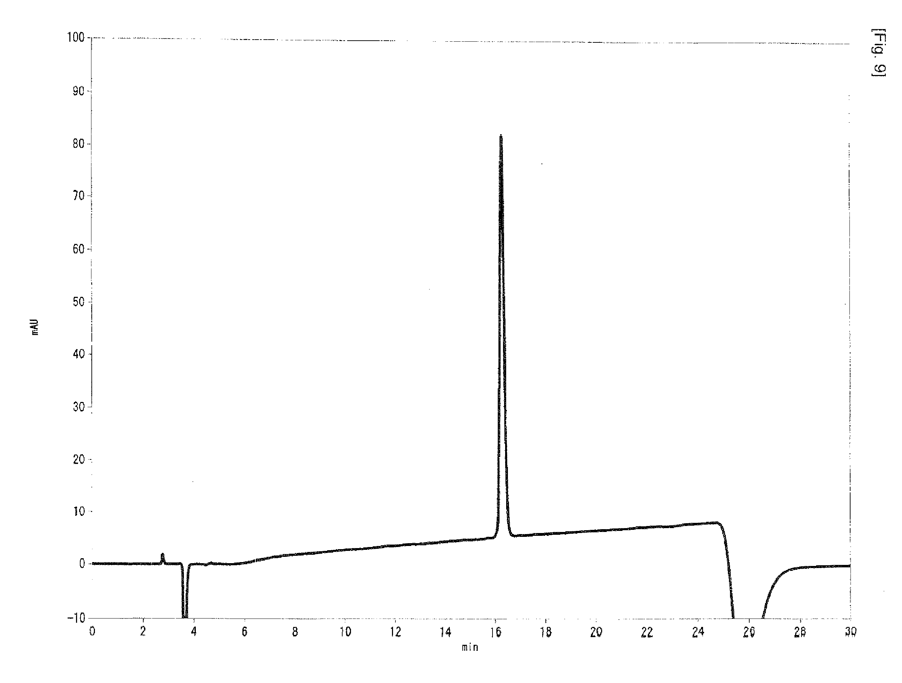

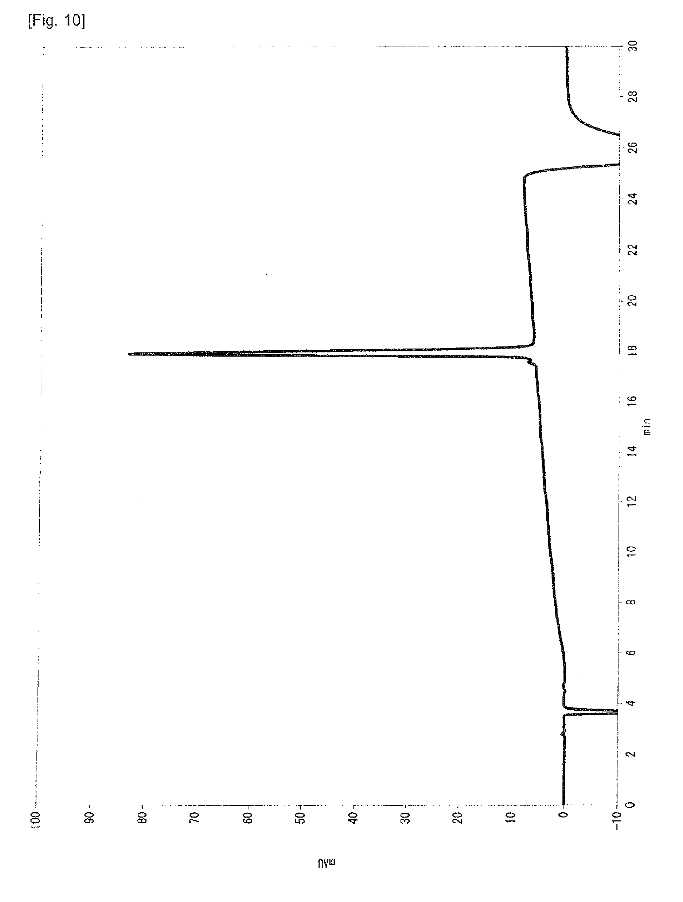

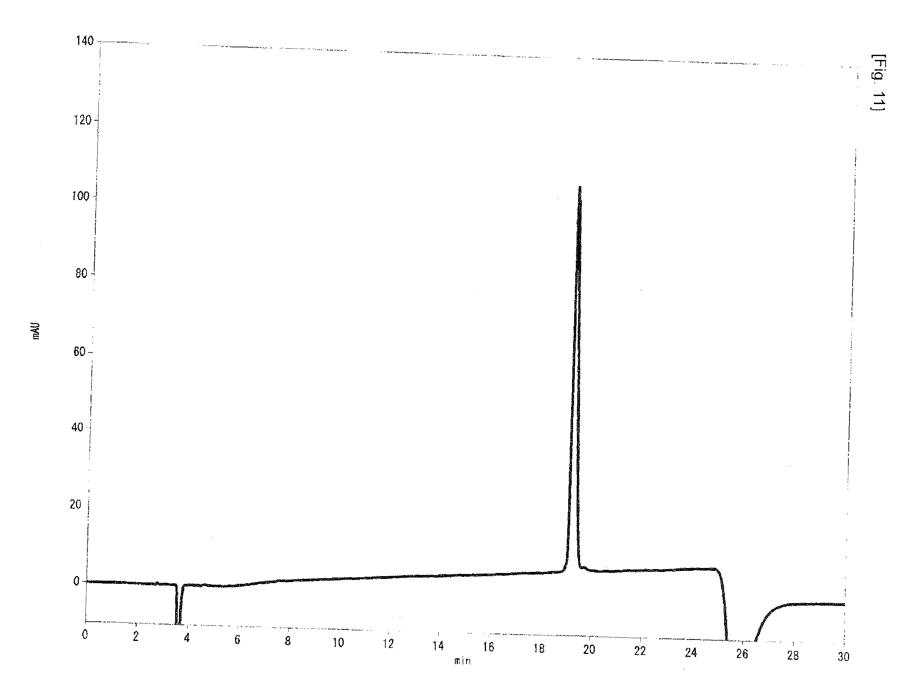

When the compounds A, B and C produced by the method described above were analyzed by a high performance liquid chromatography (HPLC) method under the conditions shown below, the area percentages (%) of the compounds A, B and C were 99.0%, 98.2% and 98.3%, respectively. The area percentage (%) was determined by designating the sum of the peak areas of the respective compounds obtained from a chromatogram for the retention time in the range of 6 minutes to 22 minutes, as 100, and calculating the ratios of peak areas of the respective components with respect to the sum of peak areas. The HPLC chromatograms of the compound A to the compound C are shown in FIG. 9 to FIG. 11.
1) Column: Unison US-C18 250-4.6 mm (manufactured by Imtakt Corp.)
2) Mobile phase: 1-7% aqueous MeCN solution (containing 0.05% TFA) (0 to 20 minutes)
3) Flow rate: 1.0 ml/min
4) Detection: UV, 210 nm
5) Column temperature: 50°C
6) Retention time: compound A 16.3 minutes, compound B 17.9 minutes, compound C 19.2 minutes
(Reference Document: Chemistry Handbook: Applied Chemistry, 6th Edition (Maruzen Corp.), Chapter 8 Analysis, Measurement, Management, p. 342-346)

### Example 2

The α-amylase inhibitory activity of the compound of the invention was verified by the following method.

### (1) Experiment method

The solutions of pancreatic α-amylases of mouse, rat, dog and monkey were prepared from the pancreases of an ICR mouse (male, 8 weeks old, purchased from SLC Japan, Inc.), an SD rat (male, 8 weeks old, purchased from Charles River Laboratories Japan, Inc.), a beagle dog (male, 35 months old, purchased from Narc Corp.), and a cynomolgus monkey (male, 10 years old, purchased from CLEA Japan, Inc.). The solutions of α-amylases from human saliva and pancreas were prepared from the enzymes purchased from Sigma-Aldrich Co. These α-amylase solutions were all diluted to 800 U/mL using an assay buffer (48 mM NaCl, 5.4 mM KCl, 28 mM Na₂HPO₄, 43 mM NaH₂PO₄, 35 mM mannitol, pH = 7.0). The various α-amylase solutions (20 U, 25 µL) and the compounds dissolved in the assay buffer (25 µL) were added to a 96-well microplate, and the microplate was incubated at 37°C for 10 minutes. Subsequently, a starch solution (5 mg/mL, 50 µL) was added thereto, and the microplate was incubated at 37°C for 10 minutes. A 0.33 M perchloric acid solution (50 µL) was added to the microplate to terminate the enzymatic reaction, and then a 0.01 M iodine solution (50 µL) was added thereto to color the solutions. The absorbance (660 nm) of each of the solutions was measured. The concentration of the compound inhibiting 50% of the α-amylase activity was calculated as the IC₅₀ value.

### (2) Results

The compounds of the invention have an inhibitory activity against all kinds of the α-amylases examined. The inhibitory activity (IC₅₀ value) of the compounds A, B and C against the mouse pancreatic α-amylase were 1.90 nM, 2.06 nM and 1.73 nM, respectively; the inhibitory activity (IC₅₀ value) against the rat pancreatic α-amylase were 2.01 nM, 2.12 nM and 1.95 nM, respectively; the inhibitory activity (IC₅₀ value) against the dog pancreatic α-amylase were 2.06 nM, 2.38 nM and 2.07 nM, respectively; the inhibitory activity (IC₅₀ value) against the monkey pancreatic α-amylase were 2.14 nM, 1.90 nM and 2.02 nM, respectively; the inhibitory activity (IC₅₀ value) against the human saliva α-amylase were 2.20 nM, 1.87 nM and 1.99 nM, respectively; and the inhibitory activity (IC₅₀ value) against the human pancreatic α-amylase were 2.02 nM, 2.11 nM and 2.21 nM, respectively.

### Example 3

The oral activity of the compounds of the invention was verified by the following method.

### (1) Experiment method

Male ICR (normal) mice (6 weeks old, purchased from SLC Japan, Inc.) were used for the animal. The compound was prepared into a solution using a 0.5% methylcellulose solution. Blood was collected from a mouse which had been fasted overnight, for the measurement of the blood glucose level and the blood plasma insulin level, and the mouse was orally administered with a solvent or the compound A (0.3, 1, 3, 10 mg/kg), immediately followed by oral administration of a carbohydrate solution (75 mg/mL starch, 25 mg/mL sucrose, 20 mL/kg). Subsequently, blood was collected 0.25, 0.5 and 1 hour after the oral administration, for the measurement of the blood plasma insulin level, and blood was collected 0.5, 1 and 2 hours after the oral administration, for the measurement of the blood glucose level.
The blood glucose level was measured by using Glucose CII-test Wako reagent (Wako Pure Chemical Industry, Ltd.), and the blood plasma insulin concentration was measured by using a Mouse Insulin ELISA Kit (Shibayagi Co., Ltd.). The test results are indicated in the average ± standard error.
The area-under-curve (AUC) of the blood glucose level-time curve was calculated from the blood glucose level for the time period up to 2 hours from the administration of the compound, and the AUC of the plasma insulin level-time curve was calculated from the blood plasma insulin level for the time period up to 1 hour from the administration of the compound. A statistical analysis was performed between the solvent-administered group and the compound A-administered group by using Dunnett's multiple range test, and a critical rate of less than 5% was defined as significant.

### (2) Results

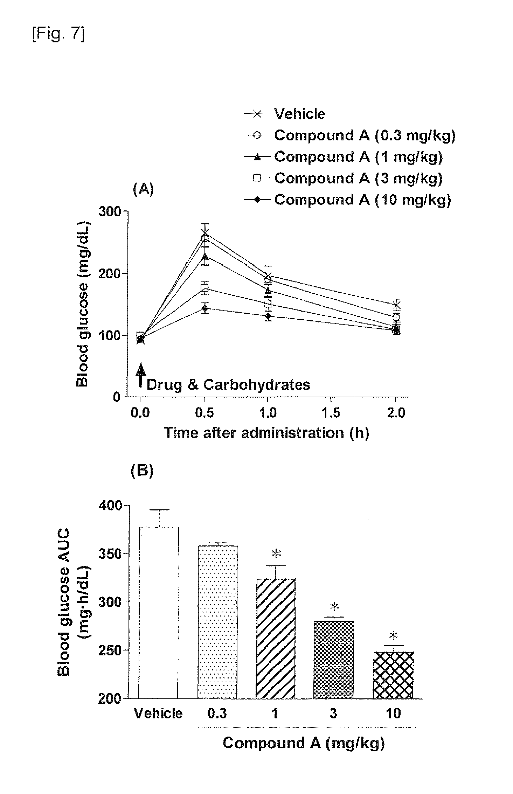

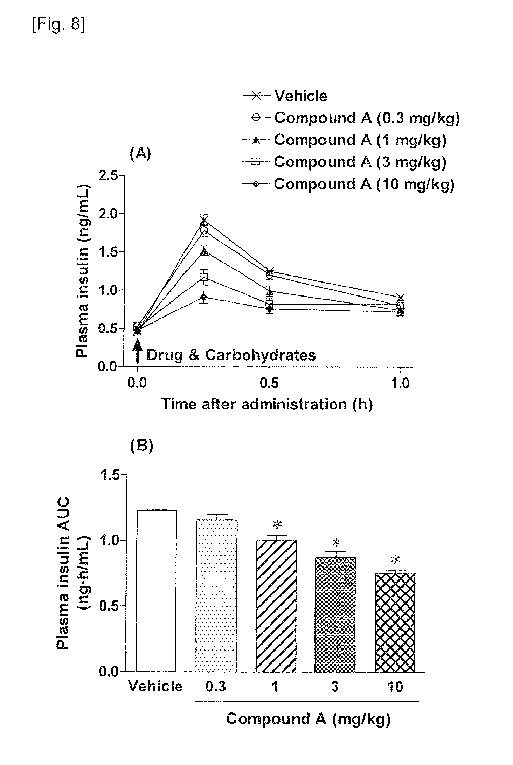

A dose-dependent blood glucose elevation suppressive action was recognized by the oral administration of the compound A (0.3 to 10 mg/kg), and the action was significant for a dose of 1 mg/kg or greater (FIG. 7). This time, a dose-dependent, significant plasma insulin level lowering action was also recognized (FIG. 8).

### Example 4

The disaccharide hydrolase inhibitory activity of the compounds of the invention was verified by the following method.

### (1) Experiment method

The brush border cell samples produced from the small intestines of an ICR mouse, an SD rat, a beagle dog and a cynomolgus monkey, and a human small intestinal microsome sample (BD Biosciences; Lot 36869) were used as various disaccharide hydrolase solutions. These disaccharide hydrolase solutions and the compounds were all prepared using a phosphate buffer (NaCl 48 mM, KCl 5.4 mM, Na₂HPO₄ 28 mM, NaH₂PO₄ 43 mM, and mannitol 35 mM, pH = 6.0).
Evaluation on mouse, rat, dog and monkey disaccharide hydrolase inhibitory activity: The disaccharide hydrolase solutions (10 mg/mL, 40 µL) and compound solutions (20 µL) were added to a 96-well microplate, and the microplate was incubated for 10 minutes at 37°C. Subsequently, substrates for the disaccharide hydrolases (sucrase: 100 mM sucrose, maltase: 100 mM maltose, isomaltase: 100 mM isomaltose, lactase: 100 mM lactose, trehalase: 100 mM trehalose, 40 µL) were added to the microplate, and the microplate was incubated for 30 minutes at 37°C. A 0.04 M perchloric acid solution (100 µL) was added to the microplate to terminate the reaction, and then the solutions were centrifuged (2,000 rpm, 15 minutes). The glucose concentrations in the supernatants were measured using Glucose CII-test Wako reagent (Wako Pure Chemical Industry, Ltd.). The disaccharide hydrolase inhibitory activity was calculated relative to non-enzyme-added samples (0%) and non-compound-added samples (100%).
Evaluation on human disaccharide hydrolase inhibitory activity: The disaccharide hydrolase solutions (0.1 mg/mL, 20 µL) and compound solutions (10 µL) were added to a 96- well microplate, and the microplate was incubated for 10 minutes at 37°C. Subsequently, substrates for the disaccharide hydrolases (sucrase: 100 mM sucrose, maltase: 100 mM maltose, isomaltase: 100 mM isomaltose, lactase: 100 mM lactose, trehalase: 100 mM trehalose, 20 µL) were added to the microplate, and the microplate was incubated for 30 minutes at 37°C. A 0.04 M perchloric acid solution (100 µL) was added to the microplate to terminate the reaction, and then the solutions were centrifuged (2,000 rpm, 15 minutes). The glucose concentrations in the supernatants were measured using Glucose CII-test Wako reagent (Wako Pure Chemical Industry, Ltd.). The disaccharide hydrolase inhibitory activity was calculated relative to non-enzyme-added samples (0%) and non-compound-added samples (100%).

### (2) Results

The compounds of the invention did not exhibit an inhibitory activity against any of the disaccharide hydrolases (sucrase, maltase, isomaltase, lactase and trehalase) brought under test (IC₅₀ > 10 µM).

As a result of the tests described above, it was confirmed that the compound of formula (I) has an α-amylase inhibitory action, and a blood glucose level and blood plasma insulin level lowering action, and it was confirmed that the compound can be used for the treatment of diabetes and the like.

### Industrial Applicability

The compound of formula (I) or a salt thereof has an α-amylase inhibitory action and can be used as a prophylactic or therapeutic agent for diabetes, obesity, NASH (non-alcoholic steatohepatitis) and the like. The compound or a salt thereof is also expected to be able to inhibit the absorption of glucose without causing symptoms in the digestive system such as diarrhea, unlike the glucosidase inhibitors.

### Sequence Listing Free Text

The 16S rDNA partial base sequence of *Streptomyces* sp. strain 6982 is shown in the Sequence Listing.

## Claims

1. A compound of formula (I) or a salt thereof: 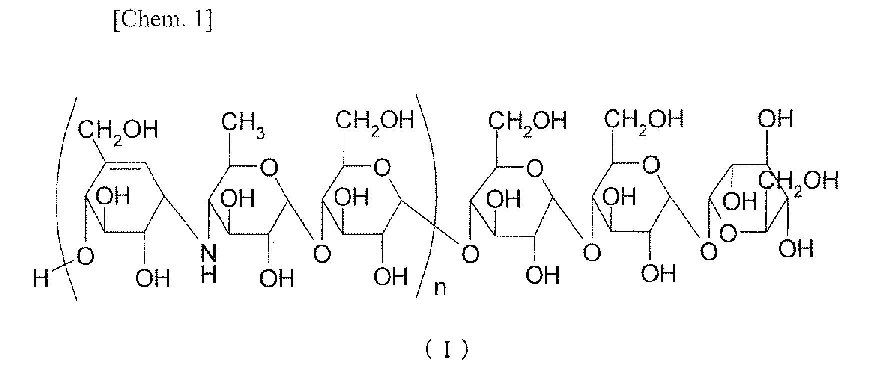 wherein n is 4 to 6.

2. A compound having an α-amylase inhibitory activity and having a molecular formula of C₉₄H₁₅₆N₄O₆₄, C₁₁₃H₁₈₇N₅O₇₆ or C₁₃₂H₂₁₈N₆O₈₈ (provided that those compounds having a reducing sugar structure at the terminals are excluded), or a salt thereof, the compound being obtainable by culturing a microorganism belonging to the genus *Streptomyces* that produces a compound having an α-amylase inhibitory activity or a salt thereof in a medium, and recovering the compound or a salt thereof from the culture medium.

3. The compound (provided that those compounds having a reducing sugar structure at the terminals are excluded), or a salt thereof according to claim 2, wherein the microorganism belonging to the genus *Streptomyces* is *Streptomyces* sp. strain 6982 (FERM BP-10802).

4. *Streptomyces* sp. strain 6982, which is a actinomycetes of the genus *Streptomyces* deposited under the Accession No. FERM BP-10802, or a mutant strain thereof.

5. A composition comprising the compound of formula (I) which has been purified or isolated from a culture medium of a microorganism of the genus *Streptomyces,* or a salt thereof.

6. The composition according to claim 5, wherein the microorganism of the genus *Streptomyces* is *Streptomyces* sp. strain 6982 (FERM BP-10802).

7. A composition comprising the compound of formula (I) or a salt thereof at a proportion of about 80% or more in terms of the area percentage as measured by chromatography.

8. The composition according to claim 5, comprising the compound of formula (I) or a salt thereof at a proportion of about 80% or more in terms of the area percentage as measured by chromatography.

9. The composition according to claim 6, comprising the compound of formula (I) or a salt thereof at a proportion of about 80% or more in terms of the area percentage as measured by chromatography.

10. A process for production of a compound having an α-amylase inhibitory activity and having a molecular formula of C₉₄H₁₅₆N₄O₆₄, C₁₁₃H₁₈₇N₅O₇₆ or C₁₃₂H₂₁₈N₆O₈₈ (provided that those compounds having a reducing sugar structure at the terminals are excluded), or a salt thereof, the process comprising culturing a microorganism belonging to the genus *Streptomyces* that produces a compound having an α-amylase inhibitory activity in a medium, and recovering the compound from the culture medium.

11. The process for production according to claim 10, wherein the microorganism belonging to the genus *Streptomyces* is *Streptomyces* sp. strain 6982 (FERM BP-10802).

12. A process for production of a compound of formula (I) or a salt thereof, the process comprising purifying or isolating from a culture medium of a microorganism of the genus *Streptomyces.*

13. The process for production according to claim 12, wherein the microorganism belonging to the genus *Streptomyces* is *Streptomyces* sp. strain 6982 (FERM BP-10802).

14. A pharmaceutical composition comprising the compound or a salt thereof according to claim 1, and a pharmaceutically acceptable excipient.

15. A pharmaceutical composition for the prevention or treatment of diabetes, comprising the compound or a salt thereof according to claim 1.

16. Use of the compound or a salt thereof according to claim 1, for the production of a pharmaceutical composition for the prevention or treatment of diabetes.

17. Use of the compound or a salt thereof according to claim 1, for the prevention or treatment of diabetes.

18. A method for the prevention or treatment of diabetes, the method comprising administering an effective amount of the compound or a salt thereof according to claim 1 to a patient.
